# EUROPEAN PATENT APPLICATION

(11) **EP 4 710 917 A1**
(43) Date of publication of application: **18.03.2026**
(21) Application number: 24803525.5
(22) Date of filing: 09.05.2024
(51) Int. Cl.: A61K 8/9728, A61Q 1/02, A61Q 17/04, A61Q 19/00

(54) **COSMETIC COMPOSITION USING YEAST CELL WALL**

(30) Priority: 09.05.2023 JP 2023077515
(71) Applicant: Mitsubishi Corporation Life Sciences Limited, Tokyo 100-0006 (JP)
(72) Inventor: ITO Masayuki, Tokyo 100-0006 (JP); MATSUMOTO Takunori, Tokyo 100-0006 (JP); FUKANO Kazuhiro, Tokyo 100-0006 (JP)
(74) Representative: Müller-Boré & Partner Patentanwälte PartG mbB
(86) International application number: PCT/JP2024/017283
(87) International publication number: WO 2024/232416

(57) **Abstract**

[Problem] To provide a material which utilizes a yeast-derived cell wall, has a skin wrinkle improving effect, and can be used as a feeling improver when applied to the skin.

[Solution] As a result of intensive studies, the present inventors have found that, by using a yeast cell wall-containing composition having a yeast cell wall structure, a cosmetic composition having a skin wrinkle improving effect, a moisture retaining effect and a skin barrier effect can be provided, and that the cosmetic composition can also be used as a feeling improver. The present invention is completed based on these findings. Furthermore, yeast cell wall-containing composition according to the present invention can be extracted from yeast that has been used in foods. That is, the composition is derived from a microorganism capable of being used in foods, and therefore can be used safely.

## Description

### Technical Field

The present invention relates to a cosmetic composition containing a yeast-derived material.

### Background Art

For the purpose of maintaining healthy skin and care, cosmetics such as a lotion and an emulsion, and external medicines are used. In skin improvement and the like, from the viewpoint of safety and the like, naturally derived materials may be used, and microorganism-derived materials such as lactic acid bacteria and yeasts are utilized.

For example, β-glucans derived from yeasts and basidiomycetes, which have effects such as anti-aging, moisturizing, and skin beautifying effects on aged skin, have been reported (Patent Literature 1).

### Citation List

### Patent Literature

Patent Literature 1: JP 2005-15348 A

### Summary of Invention

### Technical Problem

An object of the present invention is to utilize a yeast-derived cell wall to provide a material having a skin wrinkle improving effect.

### Solution to Problem

As a result of intensive studies, the present inventors have found that the above-described problems are solved by using a yeast cell wall, and have completed the present invention.

That is, the present invention relates to:
(1) a moisturizing external composition for skin including a yeast cell wall-containing composition maintaining a yeast cell wall shape;
(2) a wrinkle improving external composition for skin including a yeast cell wall-containing composition maintaining a yeast cell wall shape;
(3) an external composition for skin having moisture retention persistence and occlusive property, including a yeast cell wall-containing composition maintaining a yeast cell wall shape;
(4) an external composition for skin having sebum adsorption, including a yeast cell wall-containing composition maintaining a yeast cell wall shape;
(5) A white floating suppression-type external composition for skin including a yeast cell wall-containing composition maintaining a yeast cell wall shape;
(6) an external composition for skin including a yeast cell wall-containing composition maintaining a yeast cell wall shape of genus saccharomyces;
(7) an external composition for skin, wherein an average particle diameter of a yeast cell wall maintaining one yeast cell wall shape selected from (1) to (6) is 5 to 10 µm; and
(8) a method for sustaining occlusive property of skin and impartment of moisture to the skin using an external composition for skin containing a yeast cell wall-containing composition maintaining a yeast cell wall shape.

### Advantageous Effects of Invention

According to the present invention, a cosmetic composition having a skin wrinkle improving effect, a moisture retaining effect, and a skin barrier effect can be provided, and the cosmetic composition can also be used as a feeling improver.

Furthermore, the yeast cell wall-containing composition according to the present invention can be extracted from yeast that has been used in foods. That is, the composition is derived from a microorganism capable of being used in foods, and therefore can be used safely.

### Description of Embodiments

The present invention utilizes a yeast cell wall-containing composition maintaining a yeast cell wall shape. In the present application, "maintaining a yeast cell wall shape" means that the structure of a yeast cell wall is only required to be maintained even partially, and a yeast cell body shape is not required to be constituted. The "yeast cell wall-containing composition maintaining a yeast cell wall shape" includes those in which the yeast cell wall retains the cell body shape, those in which a part of the cell wall is deficient from the yeast cell body shape, and those in which the structure of the yeast cell wall is maintained even in whole or in part.

As a yeast to be used for the production of the composition containing a yeast cell wall of the present invention, any yeast may be used as long as it is a yeast belonging to a taxonomic yeast. It is preferable to use yeasts generally used in food production and the like. Specific examples thereof include Saccharomyces cerevisiae, Saccharomyces pastrianus, Saccharomyces rouxii, and Saccharomyces carlsbergensis, which are yeasts belonging to the genus Saccharomyces, and Candida utilis, Candida tropicalis, Candida lipolytica, and Candida flaveri, which are yeasts belonging to the genus Candida. Preferably, a yeast belonging to the genus Saccharomyces such as Saccharomyces cerevisiae, Saccharomyces pastrianus, Saccharomyces rouxii, or Saccharomyces carlsbergensis is used, and more preferably Saccharomyces cerevisiae or Saccharomyces pastrianus is used. These yeasts may be used alone or in combination of two or more selected from the above yeasts. Among them, a beer yeast is particularly preferable. When the beer yeast is used, in order to remove bitterness caused by beer, it is preferable to use yeast cell bodies subjected to a debittering treatment such as washing with an alkaline aqueous solution. A method well known to those skilled in the art can be used for the debittering treatment, and there is no particular limitation. Specifically, it can be performed by the method described in JP 55-162983 A.

The composition containing a cell wall used in the present invention can be prepared by a method well known to those skilled in the art. In general, a yeast cell wall fraction used in the present invention can be prepared by destroying yeast cell bodies and excluding intracellular ingredients.

Hereinafter, a method for preparing a composition containing a cell wall used in the present invention will be exemplified.

Yeast cell bodies are digested with an enzyme. The digestion treatment of the yeast cell bodies may be performed using an autolysis method using an enzyme present in the yeast cell bodies, or may be performed using an enzyme addition method in which an enzyme such as protease, nuclease, β-glucanase, esterase or lipase is added from the outside. Furthermore, the digestion treatment may be performed using an autolysis method and an enzyme addition method in combination. The digestion treatment to be used may be a treatment other than the above, and any method may be used as long as it is a method generally used in preparing a yeast extract. Before the digestion treatment, a pretreatment involving the physical destruction of the cell wall may be performed by a high pressure homogenizer or the like for the purpose of reducing a digestion treatment time with the enzyme as necessary. The pretreatment using the high pressure homogenizer can be performed, for example, under a pressure of 100 to 1000 kg/cm2. After the digestion treatment, the soluble cell ingredients of the enzyme-treated product are removed by centrifugation or the like. Then, a pellet is treated with an acidic aqueous solution, and a yeast cell body residue obtained as an insoluble ingredient is recovered by centrifugation or the like, so that the composition containing a cell wall of the present invention can be obtained. As the acidic aqueous solution, an acid of 0.01 to 2 N, and preferably 0.1 to 0.5 N, such as hydrochloric acid, sulfuric acid, or nitric acid, can be used. The acid treatment can be preferably performed under heating to 50°C or higher, and more preferably performed under heating to 80°C or higher.

In the present invention, a yeast extract extraction residue generated during the production of a known yeast extract can also be used. The obtained composition containing a cell wall can be appropriately suspended in a solvent such as water or ethanol for use. The composition containing a cell wall can also be used in a solid form or in a powder form after drying.

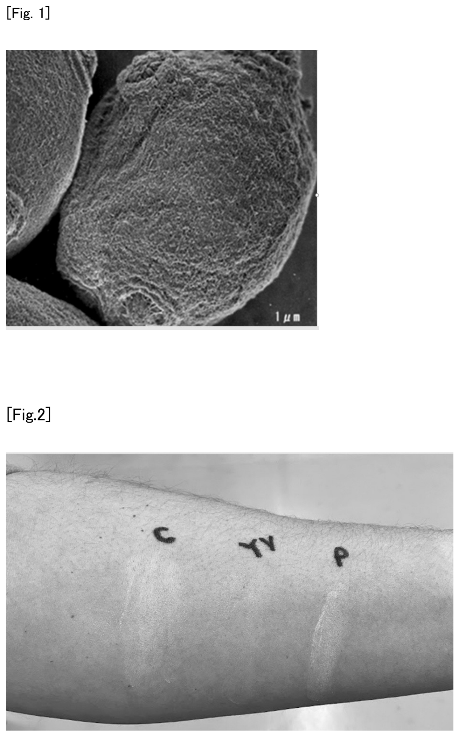

The composition containing a cell wall thus adjusted maintains the form of the cell wall of the yeast. (Fig. 1) The composition containing a cell wall of the present invention has an average particle size of 5 to 15 µm. The average particle size of the present invention was measured by a wet method using a laser diffraction particle size distribution analyzer (SALD-300V, manufactured by Shimadzu Corporation), and a median size was defined as the particle size.

The composition containing a cell wall of the present invention contains a dietary fiber containing β-glucan. The content of the dietary fiber in the composition of the present invention is 40% by weight or more, preferably 45% by weight or more, and more preferably 50% by weight or more in the solid content. β-glucan is contained in the dietary fiber in an amount of 30% by weight or more, preferably 40% by weight or more, and more preferably 45% by weight or more.

The composition containing a cell wall of the present invention can be used as a cosmetic composition. As a use method, for example, the product of the present invention can be blended in the cosmetic composition for use. The blending amount of the cell wall-containing composition of the present invention in the cosmetic composition is arbitrary, and may be a blending amount that can be appropriately selected by those skilled in the art. In order to make the wrinkle preventing effect, wrinkle improving effect, moisture retention persistence and occlusive property (barrier property), and white floating suppressing effect of the present invention more excellent, it is desirable that the cell wall-containing composition of the present invention is contained in the cosmetic composition in an amount of 0.01 to 30% by weight, preferably 0.05 to 15% by weight, and more preferably 0.1 to 10% by weight.

In the cosmetic composition using the present invention, other than the present invention, general ingredients that can be blended in the cosmetic composition can be blended. Examples thereof include various active ingredients such as oils, alcohols, ethers, surfactants, sequestering agents, pearl agents, amino acids, organic amines, water-soluble polymers, polymer emulsions, pH adjusters, vitamins, antioxidants, preservatives, water-soluble polymers, fragrances, ultraviolet absorbers, and ultraviolet ray blockers. Various vitamins, whitening agents, amino acids, anti-inflammatory agents, and the like to be blended in cosmetic materials can also be blended as medicinal ingredients.

Examples of the types of the cosmetic materials of the present invention include skin external agents such as basic cosmetic materials, makeup cosmetic materials, aromatic cosmetic materials, body cosmetic materials, and ointments. These can be produced according to a usual method. Preferable examples of the dosage forms of the cosmetic materials or skin external agents of the present invention include emulsifying type cosmetic materials such as an oil-in-water (O/W) type, a water-in-oil (W/O) type, a W/O/W type, and an O/W/O type; and dosage forms such as an oily cosmetic material, a solid cosmetic material, a liquid cosmetic material, a kneaded cosmetic material, a stick-like cosmetic material, a volatile oil type cosmetic material, a powdery cosmetic material, a jelly cosmetic material, a gel cosmetic material, a paste cosmetic material, an emulsion polymer type cosmetic material, a sheet cosmetic material, a mist cosmetic material, and a spray type cosmetic material.

Hereinafter, the present invention will be further described in detail with reference to Examples, however the present invention is not limited to these Examples.

### Example 1

### (Production of Composition of Present Invention)

### Example 1

A beer yeast slurry obtained by beer production was obtained, and a muddy live yeast obtained by centrifugation at 4500 rpm for 10 minutes was suspended in water so as to have a solid content of 5% by weight. The suspended matter was autolyzed under reaction conditions at 50°C for 17 hours, and then recentrifuged to obtain an autolyzed residue from which a soluble intracellular ingredient was removed, as a cell wall-containing composition. The average particle size of the cell wall-containing composition was 5.7 um, a dietary fiber in the solid content was 51% by weight, and the content of β-glucan in the dietary fiber was 45% by weight. The composition was subjected to the following test as Production Example 1.

### (Human Test)

A test product was applied to 20 subjects (females, mean age: 49.3) twice in the morning and evening, and the skins were subjected to various measurements before the start of the test and after 28 days. In the test, an emulsion containing 3% of the composition of Production Example 1 was adjusted by a conventional method according to the formulation in Table 1 for use. A coating method and an evaluation method are also shown below.

**[Table 1]**

| DISPLAY NAME | RAW MATERIAL NAME | % |
|---|---|---|
| BEHENYL ALCOHOL, POLYGLYCERYL-10 PENTASTEARATE, Na STEAROYL LACTYLATE | NIKKOL NICOMULES 41S | 2.00 |
| SQUALANE | HAI OLIVE SQUALANE | 5.00 |
| PURIFIED WATER | WATER | APPROPRIATE AMOUNT |
| LECITHIN, LYSOLECITHIN, LYSOPHOSPHATIDIC ACID | LPA (NIKKO CHEMICAL) | 0.10 |
| BG | HAI HAISUGARCANE BG | 10.00 |
| COMPOSITION OF PRODUCTION EXAMPLE | YEAST CELL WALL | 3.00 |
| PENTYLENE GLYCOL | DIOL PD-V | 4.00 |
| GLYCERIN | GLYCERIN | 4.00 |
| HYDROXYETHYL CELLULOSE, WATER | DAICEL HEC 1.0%soln | 5.00 |
| (ACRYLATE/ALKYL ACRYLATE (C10-30)) CROSS POLYMER, WATER | PEMULEN TR2 2%Soln | 20.00 |
| PURIFIED WATER | WATER | 1.23 |
| Na HYDROXIDE | Na HYDROXIDE | 0.137 |
| ETHYLHEXYLGLYCERIN | ETHYLHEXYLGLYCERIN | 0.10 |
| PHENOXYETHANOL | PHENOXYETHANOL | 0.50 |
| | IN TOTAL MOUNT (%) | 100 |

### -Coating Method

After makeup removing and face washing, the skin was conditioned with a lotion that is usually used, and then 4 pumps of the test product were taken into the hand and spread over the entire face. 4 pumps of the test product were taken into the hand again and applied to the entire face again (face washing → lotion → first test product → second test product).

### -Evaluation Method

After the face washing, measurement was performed with various measuring instruments after acclimatization in a thermo-hygrostat chamber (room temperature: 22±2°C, humidity: 50±10%) for 20 minutes.

The results are illustrated in Table 2. A stratum corneum water content was about 1.3 times, indicating a significant increase. Also in the texture, a significant increase in a texture volume ratio was observed by replica analysis. A wrinkle improving effect was measured using VISIA, and it was shown that the number of wrinkles was significantly reduced after the test.

**[Table 2]**

| SUBJECTS | 20 FEMALES, MEAN AGE: 49.3±8.7 |
|---|---|
| WATER CONTENT (MEASUREMENT OF STRATUM CORNEUM WATER CONTENT BY CORNEOMETER) | SIGNIFICANT INCREASE |
| | 46.7±7.4→59.5±9.1 |
| AMOUNT OF MELANIN (MEASUREMENT OF COLOR OF CHEEK BY MEXAMETER) | SIGNIFICANT DECREASE |
| | 155.3±24.3→142.1±21.5 |
| TEXTURE ANALYSIS (REPLICA/IMAGE ANALYSIS) | SIGNIFICANT INCREASE IN TEXTURE VOLUME RATIO |
| | 20.5±11.4m³/mm²/100→26.8±13.5µm³/mm²/100 |
| WRINKLE IMPROVEMENT (IMAGING BY VISIA) | SIGNIFICANTLY LOW VALUE |
| | 120.0±53.6 WRINKLES→94.0±43.0 WRINKLES |

### (Sensory Test)

The composition of Production Example 1 was blended in a sunscreen agent, a liquid foundation containing a pigment, or the like by a conventional method, and skin touch and the like were evaluated by sensory evaluation as compared with those in which the product of the present invention was not blended. As a result, sensory evaluators evaluated that the texture was improved to a very smooth texture. It was found that the yeast cell wall can also be used as a feeling improver.

From the results of the sensory test, the application of the feeling improver to the skin was further tested. 3% of the composition of Production Example 1 was blended in a sunscreen formulation containing a solid pigment/particles (titanium oxide). As a comparison object, a control (unblended product) and an acrylic resin (PMMA) blended product generally used in cosmetics were adjusted in the same manner. Each formulation was applied to the arm and then spread using the finger pad.

### (Description of symbols in Fig. 2, C: control, YV: product of the present invention, P: PMMA)

The results are illustrated in Fig. 2. In the blended product of Production Example 1, white floating was reduced as compared with the unblended sunscreen. Meanwhile, in PMMA having a similar particle size, white floating could not be prevented, and the skin touch was also improved as in the sensory evaluation in the preceding paragraph.

### (Persistence of Moisture Retention)

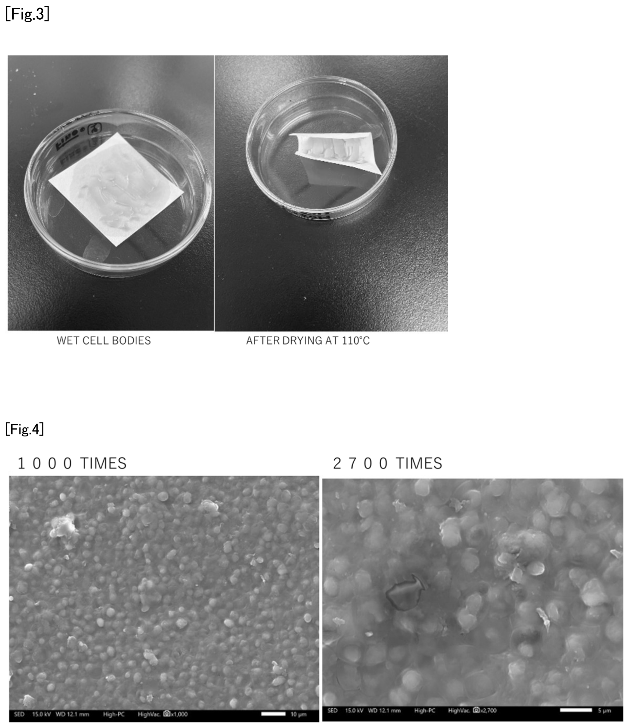

The composition of Production Example 1 was thinly spread on a filter paper, and the moisture retention was verified (Fig. 3).
Initial value: undried composition of Production Example 1 (moisture was removed by centrifugal recovery): 0.2891 g
Dried at 40°C: 0.0340 g
Dried at 110°C: 0.0237 g

Assuming complete drying at 110°C, 0.0340-0.0237=0.0103 g of water was retained. A moisture retention ratio was 0.0103÷0.237*100=43%.

Investigations were made as to whether the product of the present invention achieved both moisture retention and skin barrier property (occlusive property).
Sample: 1% solution of composition of Production

### Example 1

### Electron microscopy method:

### <Sample Pretreatment>

- A sample solution was poured into a petri dish, and then stored in a freezer at -80°C for 30 minutes or more.
- 24 hours, vacuum lyophilization

### <Imaging Conditions>

Sample adhesion method: adhesion of 0.7 to 1.5 mg/cm2 to conductive carbon double-sided tape
Antistatic conditions: coating material_Au, film thickness to be formed_5 nm, device_JEC-3000FC used
Measurement conditions: voltage_15 kV, mode_SED_high vacuum, device_JCM-7000

The results revealed that the product of the present invention was densely present also in a cosmetic application concentration and amount as shown in Fig. 4. The skin is damaged by external factors such as PM2.5 and pollen which are air pollutants, but it is considered that this dense structure formed by the product of the present invention can protect the skin from these external factors.

Furthermore, it was verified whether or not the skin had occlusive property.

Each of aqueous solutions containing 0% by weight, 1.25% by weight, 2.5% by weight,
and 3% of the product of the present invention was applied to the inner side of the forearm, and after drying, a transepidermal water loss (TEWL) was measured.

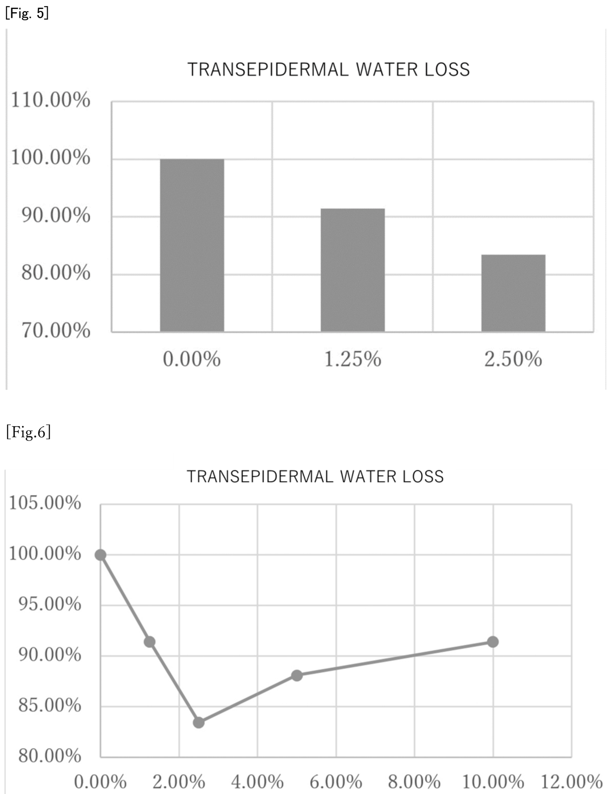

As a result, it became clear that a blocking effect of reducing the transepidermal water loss is provided. (Figs. 5 and 6)

From the above results, it was confirmed that the product of the present invention can be used as a cosmetic material that achieves both moisture retention and skin barrier property.

### Brief Description of Drawings

Fig. 1 shows an example photograph of a yeast cell wall-containing composition maintaining a yeast cell wall shape.
Fig. 2 shows a feeling improver to the skin.
Fig. 3 shows a moisture retention test using a filter paper.
Fig. 4 shows an electron micrograph.
Fig. 5 shows the verification result of the occlusive property of the skin.
Fig. 6 shows the verification result of the occlusive property of the skin.

## Claims

1. A moisturizing external composition for skin comprising a yeast cell wall-containing composition maintaining a yeast cell wall shape.

2. A wrinkle improving external composition for skin comprising a yeast cell wall-containing composition maintaining a yeast cell wall shape.

3. An external composition for skin having moisture retention persistence and occlusive property, comprising a yeast cell wall-containing composition maintaining a yeast cell wall shape.

4. An external composition for skin having sebum adsorption, comprising a yeast cell wall-containing composition maintaining a yeast cell wall shape.

5. A white floating suppression-type external composition for skin comprising a yeast cell wall-containing composition maintaining a yeast cell wall shape.

6. An external composition for skin comprising a yeast cell wall-containing composition maintaining a yeast cell wall shape of genus saccharomyces.

7. An external composition for skin, wherein an average particle diameter of a yeast cell wall maintaining one yeast cell wall shape selected from claims 1 to 6 is 5 to 10 µm.

8. A method for sustaining occlusive property of skin and impartment of moisture to the skin using an external composition for skin containing a yeast cell wall-containing composition maintaining a yeast cell wall shape.
